Europäisches Patentamt

European Patent Office

Office européen des brevets

(19)

(11) **EP 0 754 034 B1**

(12) **EUROPEAN PATENT SPECIFICATION**

(45) Date of publication and mention
of the grant of the patent:
**11.07.2001 Bulletin 2001/28**

(51) Int Cl.7: **A61K 9/51**, A61K 9/52,
A61K 9/46, A61K 9/56,
A61K 9/58, A61K 9/64

(21) Application number: **95915300.8**

(22) Date of filing: **06.04.1995**

(86) International application number:
**PCT/IE95/00025**

(87) International publication number:
**WO 95/27482 (19.10.1995 Gazette 1995/45)**

(54) **EFFERVESCENT PHARMACEUTICAL FORMULATIONS CONTAINING CONTROLLED RELEASE BIODEGRADABLE MICROCAPSULES**

PHARMAZEUTISCHE BRAUSEPRÄPARATE ENTHALTEND BIOABBAUBARE MIKROKAPSELN ZUR KONTROLIERTEN WIRKSTOFFFREISETZUNG

FORMULATIONS PHARMACEUTIQUES EFFERVESCENTES CONTENANT DES MICROGELULES BIODEGRADABLES A LIBERATION CONTROLEE

(84) Designated Contracting States:
**AT BE CH DE DK ES FR GB GR IE IT LI LU MC NL PT SE**

(30) Priority: **06.04.1994 IE 940292**

(43) Date of publication of application:
**22.01.1997 Bulletin 1997/04**

(73) Proprietor: **ELAN CORPORATION, Plc Dublin 2 (IE)**

(72) Inventors:
• **FREEMAN, Virginia**
**County Roscommon (IE)**

• **RAMTOOLA, Zebunnissa**
**Dublin 9 (IE)**

(74) Representative: **Ryan, Anne Mary et al**
**c/o Anne Ryan & Co.**
**60 Northumberland Road**
**Ballsbridge Dublin 4 (IE)**

(56) References cited:
**EP-A- 0 145 240**     **EP-A- 0 257 310**
**EP-A- 0 330 180**     **DE-A- 2 146 174**
**US-A- 4 824 675**     **US-A- 5 133 908**
**US-A- 5 178 878**

**Description**

Technical Field

This invention relates to effervescent pharmaceutical formulations containing biodegradable controlled release drug-loaded microcapsules. In particular, this invention relates to powder or tablet formulations, particularly once-daily formulations, containing a calcium antagonist, for example, nifedipine, a narcotic analgesic, for example, hydromorphone or an ACE inhibitor, for example, captopril, and the like and analogues or mixtures or combinations thereof.

Background Art

Calcium antagonists and angiotensin converting enzyme (ACE) inhibitors are widely used in patients with cardiovascular disease and are thought to have antihypertensive and vascular protective effects when administered as monotherapy or in combination. Typically, calcium antagonists are used in the treatment of hypertension and chronic stable angina; ACE inhibitors are thought to prevent the development of atherosclerosis and to reduce the number of cardiac ischemic events in patients with coronary artery disease and left ventricular dysfunction. Narcotic analgesics are widely used to provide strong relief of postoperative and chronic pain. However, their clinical usefulness is limited by the development of dependence to their action in the course of chronic treatment. Recently, it has been shown that co-administration of sublingual calcium antagonists and epidural narcotic analgesics potentiate the analgesic effect of the narcotic analgesic and suggested that co-administration might prevent the development of physical dependence on narcotics. (Pereira, *Pain*, 53: 341-355 (1993; Antkiewicz-Michaluk, *Psychopharmacology*, 111:457-64 (1993)). Typically, these drugs are orally administered in tablet/capsule formulations and, in the case of narcotic analgesics, also parenterally administered.

The efficacy of these drugs is improved when they are administered to a subject in a controlled fashion. Normal drug dosing may follow a profile of excessive-ineffective plasma levels whereby the dose initially exceeds the desired therapeutic level, and then falls to subclinical levels. However, controlled/sustained drug delivery can reduce undesirable fluctuations of drug levels, promote therapeutic benefits and minimize toxic effects. Additionally, pre-programmed delivery of a drug in the vicinity of its target cells or to its absorption sites can prevent systemic or side effects involving other tissues.

Thus, there exists a need for pharmaceutically effective, controlled release, targeted formulations containing these drugs or combinations of these drugs.

Microparticulate drug delivery systems based on biodegradable polymers, such as those formed from lactic and glycolic acid, have been widely studied for the controlled/sustained delivery of various drugs. The selection of the particular polymer(s) in light of the particular drug properties, as well as the manufacturing processes and process variables, determine the morphology and size of the microspheres, which in turn influence release characteristics for the formulation. These factors, which sometimes can negate each other, limit the possible formulations and methods for preparation of formulations suitable for controlled/sustained release, including once-daily oral administration.

Effervescent dosage forms incorporating microparticles which are adapted to provide substantially immediate release of the pharmaceutical ingredient contained in the microparticles are disclosed in US 5,178,878 and US 5,223,264.

EP-A-0257310 discloses a tablet formulation, especially a chewable tablet, formed by compressing microcapsules having a size between about 5 microns and about 400 microns, preferably between about 30 and 250 microns. The microcapsules comprise particles of active ingredient that are coated with a thin layer of a sustained release coating, such as a non-biodegradable cellulose derivative, which is chosen so as to minimise rupture of the coating during the compression process. Only long polymer length ethyl cellulose was found not to be ruptured at all during compression, even with small amounts of plasticizer.

EP-A-0145240 discloses prolonged release microcapsules and a method of producing same. The dosage forms can be for oral or parenteral administration and when intended for oral administration can be presented as, for example, a powder or a tablet. There is no disclosure of an effervescent formulation.

EP-A-0330180 discloses microspheres which comprise polylactic acid and a water soluble physiologically active substance and having a mean particle size of from about 0.01μm - 300μm. The size of the microspheres facilitates administration by the parenteral route such as by intravenous or intramuscular injection

Disclosure of the Invention

According to the invention, there is provided an effervescent pharmaceutical formulation for the sustained and controlled oral administration of a pharmaceutically effective amount of a drug selected from a calcium channel blocker, an ACE inhibitor, a narcotic analgesic or analogues or combinations thereof, said formulation comprising microcapsules having a D 50% between 100 nm and 900 nm in which the drug is entrapped in a biodegradable polymer and in which the pH of the formulation is adjusted to optimize delivery of the drug, wherein the formulation is adapted to disperse upon addition of water to form an effervescent drink.

The term microcapsule used herein includes the terms "microsphere", "microparticle", "nanosphere" and "nanoparticle". These terms do not necessarily refer to any structural relationship between the drug and the encapsulating polymer (e.g., drug encapsulated within a polymer membrane or drug and encapsulating polymer in a matrix structure). Rather, these terms simply refer to a particle (micron sized or less) in which the drug is entrapped in a polymer.

As used herein, the term "sustained/controlled release" refers to a drug release profile designed to optimize delivery of the drug to a particular site(s) of absorption such as the stomach and/or small intestine while still providing effective treatment over a predetermined extended period of time such as once-daily.

The oral formulations of this invention are designed to advantageously combine both the benefits of an effervescent or water dispersible tablet with those of a microencapsulated system. For instance, because oral effervescent formulations are easy to administer, they avoid the difficulties associated with chewing or swallowing tablets, thus increasing patient compliance. Incorporation of microcapsules in the oral formulation not only allows targeting of the release of the drug to the absorption site(s) for the drug but also provides a vehicle to control the extent of drug released with respect to time.

Further, the drug(s) is encapsulated to form microcapsules which, upon addition of water to the effervescent formulation, are dispersed to produce a fine suspension. Because this uniform suspension is formed prior to the subject ingesting the effervescent drink, the effect of food, presence of bile, pH etc. upon the dissolution of the dosage form is minimized. This is particularly useful for sparingly water soluble drugs such as nifedipine or diltiazem base. Thus, reproducibility of dosing can be improved.

Additionally, for drugs that are not well-absorbed prior to entry into the gastro-intestinal tract, ordinary immediate release effervescent formulations do not protect the drug prior to absorption, thus leading to lessened bioavailability. The addition of microencapsulation to the formula can, however, allow enough flexibility to prevent unwanted release in, for instance, the oral cavity, while promoting delivery of the drug to the targeted area for controlled/sustained release.

A burst effect is common with many microcapsules. Again, if this effect is unwanted in the oral cavity, the formulations of this invention can be designed to minimize (or, if desired, maximize) this burst effect. A particularly effective way to minimize the burst effect is to control the pH of the effervescent drink solution to optimize delivery of the or each encapsulated drug to the targeted absorption sites.

The formulation suitably is used to deliver the drug to a patient on a once-daily basis so as to achieve a therapeutic effect substantially over a 24 hour period.

The microcapsules have a D 50% between 100 nm and 900 nm, more preferably between about 200 nm and 400 nm.

Further, preferably, the drug loading of the microcapsules ranges from about 10% to 70% by weight, more preferably 20% to 50% by weight.

The invention also provides an effervescent pharmaceutical formulation for the administration of a drug selected from a calcium channel blocker, an ACE inhibitor, a narcotic analgesic or a combination thereof, the formulation comprising drug-loaded biodegradable microcapsules having a D 50% between 100 nm and 900 nm and a drug loading which ranges from about 10% to 70 % by weight.

Thus the formulations can be used to administer a selected drug to a patient on a once-daily basis so as to achieve a therapeutic effect over a substantially 24 hour period.

Suitable calcium antagonists include diltiazem, verapamil, nifedipine, nimodipine and nicardipine.

Suitable narcotic analgesics include hydromorphone, codeine sulfate, oxycodone, dihydrocodeine tartrate, oxycodeinone, morphine, fentanyl, sufentanil, oxymorphone and buprenorphine.

Suitable ACE-inhibitors include captopril, enalapril and lisinopril.

The formulations may also include a combination of two or more drugs as hereinbefore specified. Particularly preferred combinations are represented by a combination of a calcium antagonist and a narcotic analgesic. Synergism is displayed by such combinations. A suitable combination comprises nifedipine and hydromorphone.

The polymer matrix suitably comprises polylactide; polyglycolide; poly (lactic acid-co-glycolic acid); poly (e-caprolactone); poly (hydroxybutyric acid); polyortho-esters; polyacetals; polydihydropyrans; polycyanoacrylates; polypeptides; cross-linked polypeptides; and stereoisomers, racemic mixtures, co-polymers and polymer mixtures thereof.

A particularly suitable polymer matrix comprises poly-D,L-lactide.

A suitable release profile (sink conditions, such as an aqueous pH 6 solution for diltiazem) measured in accordance with the Paddle Method of U.S. Pharmacopoeia XX at 37°C and 75 rpm for the or each drug is substantially as follows:

a) 10-30% release within 2 hours after administration;

b) 30-60% release within 4 hours after administration;

c) 60-80% release within 8 hours after administration; and

d) ≥ 80% release within 24 hours after administration.

A further suitable release profile (sink conditions, such as simulated gastric fluid for captopril) measured in accordance with the Paddle Method of U.S. Pharmacopoeia XX at 37°C and 75 rpm for the or each drug is substantially as follows:

a) 10-40% release within 1 hour after administration;

b) 20-60% release within 4 hours after administration;

c) 40-80% release within 8 hours after administration; and

d) ≥ 80% release within 16 hours after administration.

A further suitable release profile (sink conditions, such as simulated gastric fluid for hydromorphone) measured in accordance with the Paddle Method of U.S. Pharmacopoeia XX at 37°C and 75 rpm for the or each drug is substantially as follows:

a) 10-50% release within 1 hour after administration;

b) 40-90% release within 4 hours after administration; and

c) >90% release within 8 hours after administration.

The effervescent formulations in accordance with the invention are suitably formulated as capsules, tablets or powders.

Thus, for convenient and effective oral effervescent administration, effective amounts of the microcapsules of the present invention can be tabletted with one or more excipient(s) or encased in capsules such as gel capsules and the like.

It will be appreciated that the pharmaceutical formulations in accordance with the invention can be used *inter alia* in a method of treating angina, hypertension, pain or the like by the administration of a formulation containing biologically effective amounts of at least one drug microencapsulated according to the invention to an animal, preferably a human, so as to provide and maintain clinically effective plasma levels of the drug throughout a substantial part of a 24 hour period.

The invention also provides a method for the manufacture of microcapsules as hereinbefore defined, which comprises the steps of;

a) dissolving or dispersing a drug and a biodegradable polymer in a solvent to form a mixture;

b) microfluidising said mixture into an external phase to form an emulsion in which the emulsion droplets have a mean diameter less than 1mm; and

c) stirring said emulsion to form microcapsules having a size (D 50%) between about 100 nm and 900 nm.

As used herein, the term "biodegradable" as applied to polymers means synthetic or natural polymers which are degradable *in vivo* either enzymatically or non-enzymatically to produce biocompatible or nontoxic by-products which can be further metabolized or excreted *via* normal physiological pathways. For instance, a range of natural and synthetic biodegradable polymers can be used to form microparticles containing a wide variety of drugs in order to achieve prolonged drug release or drug targeting. Human serum albumin (HSA), bovine serum albumin (BSA), collagen and gelatin can be used to microencapsulate the drugs. However, the cost and uncertainty of purity of natural polymers limits their use, thus focusing attention upon synthetic biodegradable polymers in which processing conditions and availability can be controlled. Examples of synthetic biodegradable polymers are those hereinbefore specified and include the following: polylactic acid (polylactide; PLA); polyglycolic acid (polyglycolide; PGA); poly (lactic acid-co-glycolic acid) (PLGA); poly (e-carpolactone); poly (hydroxybutyric acid); polyortho-esters; polyacetals; polydihydropyrans; polycyanoacrylates; polypeptides; cross-linked polypeptides; and steroisomers (i.e., D, L), racemic mixtures, co-polymers and polymer mixtures thereof. PLA is the biodegradable polymer which is most preferred.

The solvent evaporation method is one of the most common techniques for the production of PLA/PLGA microspheres. The first step in this method requires the formation of an oil-in-water emulsion (o/w emulsion). The polymer is dissolved in a volatile organic solvent followed by dissolution or dispersion of the drug in the polymer solution. This procedure is known as forming the organic phase. The resulting polymer/drug solution is stirred in a suitable suspension medium forming a droplet suspension (o/w emulsion). The suspension medium, which should not solubilize the polymer, usually contains a dispersing agent to prevent coalescence of the droplets. The suspension medium may also be referred to as the continuous phase or the external aqueous phase, the latter term being used herein.

A modification of this solvent extraction method can be used wherein the polymer and drug are first dissolved in a water miscible organic solvent such as acetonitrile and subsequently homogenized in mineral oil or light liquid paraffin to form a "w"-in-oil ("w"/o) emulsion. Another modification of this method is used, whereby a water-oil-water (w/o/w) emulsion is produced. The drug is dissolved in a small amount of water and this drug solution homogenized into a polymer/organic solvent (e.g. dichloromethane) solution. This primary water-in-oil emulsion is then emulsified with the external phase, producing the w/o/w emulsion.

Once the emulsion is formed (o/w, w/o, or w/o/w), it is subjected to solvent evaporation causing 'hardening' of the droplets (polymer solidification) and their conversion to microspheres. The emulsion is stirred continually throughout the evaporation process. During the droplet hardening process, the droplets first lose their solvent by diffusion of the solvent into the external phase followed by evaporation of the solvent from the surface. The evaporation of the solvent can take place at either atmospheric pressure or at reduced pressure. The evaporation temperature can also be varied. Solvent evaporation can be achieved in two ways: (1) interrupted evaporation in which the evaporation is stopped prior to complete elimination of the organic solvent, and the semi-solid microspheres are transferred to an emulsifier-free medium where evaporation is completed or (2) continuous evaporation in which the system is continuously agitated until all the organic solvent has completely evaporated. The solid microspheres are collected by filtration or centrifugation, washed and dried under vacuum.

Problems associated with the solvent evaporation method are agglomeration of the particles, loss of drug to the aqueous phase and the formation of drug crystals on particle surfaces. Factors affecting the quality of microspheres (i.e., drug loading, size, surface morphology), produced by the solvent evaporation method are detailed below.

In the solvent evaporation process, the organic solvent used, in addition to being able to dissolve the drug and the polymer, must be immiscible with the external phase and its boiling point must be low enough so that it will evaporate easily during the droplet hardening process. When an o/w system is used, the solvent evaporation technique works best for core materials that are insoluble in the aqueous external phase. If the core material is soluble in the external phase, it will be extracted from the microdroplets before the microsphere walls have a chance to form, resulting in lower drug loading. For instance, Bodmeier and McGinity, J. Microencap., 4:279-288 (1987) reported that water soluble drugs such as theophylline, caffeine and salicylic acid could not be entrapped by the o/w emulsion solvent evaporation method. Minimizing drug loss to the aqueous external phase can be achieved in a number of ways, such as (1) by varying the aqueous phase pH to the pH of minimal drug solubility; (2) saturating the aqueous phase with the drug; and (3) inverting the emulsification phase o/w to w/o.

The effect of using different molecular weights for a particular polymer affects the surface properties and morphology of the microspheres. For instance, high molecular weight poly-L-lactide polymers produce larger particles that tend to aggregate due to the inherent tackiness and thermoplastic nature of the polymer. Also, highly porous microspheres are produced from high molecular weight polymers whereas microspheres produced with low molecular weight polymers can be smooth and non-porous.

The diameter of the microspheres can be determined by the size of the microdroplets formed in the emulsion. The size of the microdroplets can be controlled in several ways, such as by adjusting the stirring rate, the type and the amount of emulsifier, the quantity of the aqueous phase, and the configuration of the vessel and stirrer. A general increased rate of agitation results in a finer emulsion producing smaller microspheres and preventing coalescence of smaller immature microspheres.

We have found that very fine emulsion droplets can be prepared by using a Microfluidiser (Trade Mark; Microfluidics Corp.) A Microfluidiser is a high pressure homogenizer capable of making very small size emulsion droplets (mean droplet diameter under 1mm). During microfluidisation, the emulsion is pumped through microchannels to an impingement area at high operating pressures. Cavitation and the accompanying shear and impact is responsible for the particle size reduction within the "interaction chamber".

Drug release from biodegradable systems may range from purely diffusion-controlled dissolution from a matrix system in cases in which the polymer has a slow degradation rate and the drug molecule is small, to degradation-controlled release when the drug molecule is large and its permeability in the polymer is low. With the more rapidly degrading polymers, drug release can occur through a combination of diffusion and degradation of the polymer. The possible mechanisms of drug release from biodegradable microsphere systems may be summarized as follows; (1) drug which is loosely bound to or embedded in the microsphere surface is released initially resulting in a "burst effect"; (2) depending on microsphere structure, drug may be released through the microsphere pores; (3) drug may diffuse through the polymer membrane depending on the solubility of the drug in the polymer membrane and the intrinsic properties of the polymer; (4) the drug may diffuse through the water swollen polymer barrier depending on the hydrophilicity and in turn the molecular weight of the polymer; and (5) erosion and hydrolysis of the polymer chains causes pore formation in the microsphere structure and hence affects the rate of drug release. All these possible mechanisms may together play a part in the release process depending on the morphology of the microsphere systems (which in turn is affected by manufacturing techniques), polymer composition and physiochemical properties of the drug.

Drug release from microsphere systems is dependent on the diffusivity of the drug through the polymer barrier, the solubility of the drug in the bulk phase, the size of the drug molecule and its distribution throughout the microsphere matrix. The nature of the polymer plays a major role in determining release characteristics, in particular the molecular weight of the polymer. For instance, the effect of polymer molecular weight on *in vitro* release of chlorpromazine from chlorpromazine loaded poly-DL-lactide microspheres using poly-DL-lactide in a molecular weight range from 11,000 to 21,000, in pH 7.0 aqueous phosphate buffer has been studied. Increased polymer molecular weight increased the time required for 50% release. Microspheres produced from the higher molecular weight polymers exhibited a lag time of $\geq$ 24 hours at which time the release rate increased rapidly to approximately $\leq$ 40% release.

Furthermore, two of the most important microsphere characteristics that affect drug release are drug loading and microsphere size.

Early theories which have been put forward to describe the dissolution process from matrix systems were very often based on Fick's laws of diffusion. Fick's first law of diffusion states that the flux (J) of a compound across a unit area of a predetermined reference plane is proportional to the concentration differential across that plane, the proportionality constant being the diffusivity (D). Based on Fick's first law of diffusion, Higuchi developed an equation for release of solid drug from an ointment base and later applied it to the diffusion of solid drugs dispersed in homogenous and granular matrix dosage systems as follows:

$$Q = (D(2A\text{-}Cs) \, Cs \, T)^{\frac{1}{2}} \qquad \text{(Equation 1)}$$

where

D = the diffusivity of the drug in the matrix;

A = the total amount of drug per unit volume of matrix;

Cs = the solubility of drug in the polymer matrix;

Q = amount of drug per unit area of matrix; and

T = time.

Later, using the same diffusion principle as Higuchi, Cobby *et al.* presented equations describing the release of drug from matrix tablets having either a spherical, cylindrical or biconvex shape. In each case, the three dimensional equation had the cubic form:

$$F = G_1 \, (KT^{\frac{1}{2}}) - G_2(KT^{\frac{1}{2}})^2 + G_3(KT^{\frac{1}{2}})^3 \qquad \text{(Equation 2)}$$

where

F = the fraction of drug released;

T = the elapsed time;

K = the release rate constant;

$G_1$ - $G_3$ are shape factors.

For a spherical shape $G_1$ and $G_2=3$ and $G_3 = 1$.

The fundamental parameters of the release rate constant are described by

$$K = 1/A. \; r \div D \; C_S \; (2A-eC_S)e/t.$$

where

r = initial tablet radius;

e = porosity; and

t = tortuosity.

The Prout-Tomkins' equation has been applied to the autocatalytic decomposition of solid, i.e., the drug acting as a marker of polymer degradation:

$$1n \; (c/1-c) = K \; x \; t + c \qquad\qquad \text{(Equation 3)}$$

where

c = the fraction of drug released at time t;

K = the rate constant; and

$c = K \; x \; t_{max}$ ($t_{max}$ is the time taken for 50% of the drug to be released).

The release of diltiazem base from DL-PGLA microspheres in phosphate buffer pH 7.4 has been fitted to the Prout-Tomkins' equation (Fitzgerald *et al*., Polymeric Delivery Systems, Properties and Applications, Chap. 23 (ACS Symposium Series 520) (1992)). The systems studied had drug loadings of 4.45%, 10.06% and 20.22 % and were in the <125 mm size range. In all cases, the fits obtained were good, suggesting that release from these systems was dependent on polymer degradation and dissolution.

Various methods can be used to obtain microcapsules as follow:

**Drug Dissolved in Solvent Method (Method A)**

This method is similar to the oil-in-water (o/w) emulsion solvent evaporation techniques. The drug and polymer are dissolved in a suitable solvent (such as dichloromethane) to form the oil phase. An aqueous 0.27% w/v PVA solution is suitably used as the external phase. The oil phase is emulsified into the external phase to form a droplet suspension for example by one of the following methods:

(i) Using an IKA Ultra Turrax T25 (Trade Mark) for two minutes at 8,000, 9,500, 13,500 or 24,000 rpm;

(ii) Using an M120E Microfluidiser (Microfluidics Corp.) at 10,000 psi for a specified number of cycles.

The resulting emulsion can be stirred for example with an IKA RW25 motor and 4-blade stirrer at 1,400 rpm for one hour causing the polymer droplets to lose their solvent, initially by diffusion of the solvent into the aqueous external phase and subsequently by evaporation. In this way, the polymer droplets are converted to the corresponding solid microspheres.

The microspheres are suitably collected by centrifugation (Heraeus Sepatech Biofuge 28RS) using a HFA 13.5 rotor at 13,000 rpm for 15 minutes and then dried in a vacuum oven (Gallenkamp) at 1,000 mbar for 24 hours. The microspheres are stored in airtight containers at room temperature.

## Dispersion Method (Method B)

The oil phase is formed by dispersing the drug in the polymer/solvent solution using sonication. The oil phase is emulsified into the external aqueous phase by Method A (i) or (ii). This step is followed by solvent evaporation, collection, drying and storage as outlined in Method A. Final microsphere size is dependent on drug crystal size. This limitation can be overcome by dissolving the drug in a co-solvent as outlined in Method C below.

## Co-Solvent Method (Method C).

The drug is first dissolved in the minimum amount of a suitable solvent (such as methanol). The polymer/ solvent solution is then added to the drug solution and sonicated to form the oil phase. The oil phase is emulsified into the external aqueous phase by Method A (i) or (ii). This step is followed by solvent evaporation, collection, drying and storage as outlined in Method A. As described below, diltiazem HCl was entrapped using Method C in microspheres formed from PLA with a molecular weight average of 109,000 (PLA 109K). The formulation used for a 10% w/w starting loading was as follows:

| PLA 109K | Dichloromethane | 0.9 g : 10 ml |
|---|---|---|
| Diltiazem HCl | Methanol | 0.1 g : 0.5 ml |
| Dichloromethane | 0.27% PVA | 1 ml : 10 ml |

For all methods when entrapping diltiazem, the PVA solution is suitably buffered to pH 10.0 (pH of least solubility of diltiazem in an aqueous solution) suitably using a carbonate buffer prepared as follows:

| $NaHCO_3$ | 3.915 g/l |
|---|---|
| $Na_2CO_3$ | 5.660 g/l |

## Double Emulsion Method (Method D)

This method is an adaptation of a (water-in-oil)-in-water (w/o/w) solvent evaporation technique which has previously been found to be successful in entrapping hydrophilic agents in polylactide-co-glycolide microparticles (Ogawa *et al*., Chem. Pharm Bull., 36:1502-1507 (1988)). In a modification of this method carried out by us the drug was dissolved in a 5% w/v gelatin solution (internal aqueous phase). The polymer/dichloromethane solution was added to the drug solution and emulsified at 24,000 rpm for one minute using as IKA Ultra Turrax T10. The resulting w/o emulsion was then emulsified into the 0.27% w/v PVA external aqueous phase by one of the following methods to produce the w/o/w emulsions:

(i) Using an IKA Ultra Turrax T18 at 8,000 rpm for two minutes;

(ii) Using an M120E Microfluidiser (Microfluidics Corp.) at 10,000 psi for a specified number of cycles.

The droplet hardening process was carried out by stirring the w/o/w emulsion at 1,400 rpm for 30 minutes - 1 hour (depending on the amount of solvent used) with an IKA RW25 and 4-blade stirrer. The microspheres were collected, dried and stored as outlined for Method A. For diltiazem HCl, the drug: gelatin solution ratio was 0.1 g : 0.1 ml; the polymer : solvent ratio was PLA 109K 1 g : 4 ml; and the external phase was 100 ml per 2 g total batch weight.

## *In vitro* Dissolution Studies

Dissolution studies herein were carried out in the following dissolution media:

(i) Isotonic phosphate buffer pH 7.4

| NaCl | 4.4 g/l |
|---|---|
| $NaH_2PO_4$ (anhydrous) | 1.615 g/l |
| $Na_2HPO_4$ (anhydrous) | 7.571 g/l |

(ii) Simulated gastrointestinal fluid (SGF) pH 1.2

| NaCl | 2.0 g/l |
| Concentrated HCl | 7.0 ml/l |

Stoppered dissolution flasks were filled with a measured volume of the desired dissolution medium and placed in a water bath at a constant temperature of 37∞C. After the solution had reached equilibrium, a desired amount of sample was added into the flask. The sample weight was predetermined to prevent the drug concentration in the medium from exceeding 10% of its solubility and thus maintain sink conditions. The samples were either dispersed in the dissolution media, or, if the samples were in the submicron size range, they were contained in a 14 cm length of Visking tubing tied at both ends. The dissolution flasks were shaken at 60 spm.

Brief Description of the Drawings

Fig. 1 shows the release profiles in phosphate buffer pH 7.4 of diltiazem HC1 from 10% w/w loaded PLA 109K microsphere systems prepared by Method C, $D_1$ (D 50% of 12.56 mm), $D_2$ (D 50% of 5.76 mm); $D_3$ (D 50% of 2.86 mm); and $D_4$ (D 50% of 1.41 mm);

Fig. 2 shows the release profiles in simulated gastric fluid (SGF) pH 1.2 of diltiazem HC1 from 10% w/w loaded PLA 109K microsphere systems prepared by Method C, $D_1$ (D 50% of 12.56 mm), $D_2$ (D 50% of 5.76 mm); $D_3$ (D 50% of 2.86 mm); and $D_4$ (D 50% of 1.41 mm);

Fig. 3 shows release profiles in phosphate buffer pH 7.4 of diltiazem base from PLA 109K microsphere systems prepared by Method A with the Ultra Turrax ($Db_2$-$Db_5$) 10%, 20%, 30% and 50% w/w, respectively;

Fig. 4 shows release profiles in SGF pH 1.2 of diltiazem base from PLA 109K microsphere systems prepared by Method A with the Ultra Turrax ($Db_2$-$Db_5$, 10-50% w/w respectively); and

Figs. 5, 6 and 7 show, respectively, release from 20%, 30% and 50% diltiazem base loaded PLA 109K nanospheres at pH 1.2, pH 6.0 and pH 7.4 (as prepared in Example 8).

Fig. 8 shows the diltiazem plasma levels over a period of 36 hours in humans following administration of 20%, 30%, and 50% diltiazem base loaded PLA 109K nanospheres (reference is Cardizem CD).

Fig. 9 shows release from hydromorphone loaded microcapsules encased in gel capsules into simulated gastric fluid (0.01 M Hcl).

Fig. 10 shows release from nifedipine loaded microcapsules (50% loaded R203 and 50% loaded RG 504 microspheres) and nifedipin loaded microcapsules that were mixed with lactose and tabletted prior to dissolution (30% loaded R203 and 50% loaded RG 504 microspheres); Nifed retard is shown as a control.

Diltiazem HC1 was converted to the base form when required herein by preparing a saturated diltiazem HC1 aqueous solution and adding an excess of 1 M NaOH until precipitation occurred. Recovery of the diltiazem base was followed by drying in a desiccator under vacuum for two days.

Modes for Carrying Out the Invention

The invention will be further illustrated by the following Examples.

EXAMPLE 1

An attempt was made to prepare diltiazem loaded PLA microspheres using Method A, a method involving as described above the evaporation of solvent from an o/w emulsion, the oil phase being formed by dissolving the drug and polymer in dichloromethane. This method was not suitable as diltiazem HC1 did not dissolve in dichloromethane.

EXAMPLE 2

Attempts to prepare diltiazem HC1 loaded PLA microspheres by Method B were unsuccessful. Method B also involved the evaporation of solvent from an o/w emulsion, the oil phase being formed by dispersing the drug in the polymer/solvent solution using sonication. Diltiazem HC1 crystals when observed under the light microscope were seen to be too large and irregular in shape for entrapment by this method. This finding was substantiated by particle size analysis: diltiazem HC1 had a D 10% of 19.01 mm, D 50% of 41.83 mm and a D 90% of 72.61 mm.

EXAMPLE 3

Diltiazem HC1 loaded PLA 109K microspheres were prepared using Method C, whereby the drug was first dissolved in the minimum amount of a suitable solvent (methanol). The polymer/solvent solution was then added to the drug solution and sonicated to form the oil phase. The oil phase was emulsified into the external aqueous phase (buffered to pH 10.0) using the 1KA Ultra Turrax at four different homogenization speeds. All the systems prepared by Method C had a starting loading of 10% w/w. Microsphere system $D_1$ was prepared at the lowest homogenization speed setting on the Ultra Turrax, i.e., 8,000 rpm., the microspheres produced had a drug entrapment efficiency of 86.0%. The homogenization was subsequently increased to 9,500, 13,500 and 24,000 rpm to produce microsphere systems $D_2$, $D_3$ and $D_4$ having drug entrapment efficiencies of 98.7%, 89.4% and 63.8% respectively, as shown in Table 1.

TABLE 1

| Lot No. | Speed of Homogenization Ultra Turrax rpm | Entrapment efficiency % | Yield % | D 10% mm | D 50% mm | D 90% mm | Span |
|---|---|---|---|---|---|---|---|
| $D_1$ | 8000 | 86.0 | 80.5 | 3.76 | 12.56 | 67.61 | 4.97 |
| $D_2$ | 9500 | 98.7 | 80.0 | 2.29 | 5.76 | 16.39 | 2.44 |
| $D_3$ | 13500 | 89.4 | 86.0 | 0.67 | 2.86 | 5.61 | 1.73 |
| $D_4$ | 24000 | 63.8 | 79.5 | 0.65 | 1.41 | 3.59 | 2.09 |

There was a marked decrease in the size of the microspheres with increasing homogenization speed. The D 50%, of the microspheres decreased from 12.85 mm to 1.41 mm as the homogenization speed was increased from 8,000 rpm to 24,000 rpm. There was a corresponding decrease in the D 90% from 67.61 mm to 3.59 mm and in the D 10% from 3.76 mm to 0.65 mm. This relationship was not linear when plotted on a log-log scale.

The entrapment efficiency of the microspheres was found to be dependent on the homogenization speed used. With increasing homogenization speed, there was an initial slight increase followed by a drop in efficiency with further decrease in size. This may be due to the fact that the smaller particles have a larger surface area/volume ratio resulting in greater drug loss to the aqueous external phase during processing. Percentage entrapment efficiency ranged from 63.8% to 98.7%. It was not possible to exceed 10% w/w starting loading as the amount of diltiazem needed for a starting loading greater than 10% w/w would not dissolve in 0.5 ml of methanol and in this formulation the ratio of methanol to dichloromethane should not exceed 0.5 ml to 10 ml.

SEM photomicrographs of all the microsphere systems prepared by Method C show smooth, spherical microspheres. Microspheres produced with the lowest homogenization speed 8,000 rpm were large with a D 50% value of 12.56 mm and had a wide size distribution, as indicated by the span value of 4.97. In comparison, microspheres produced with the highest homogenization speed, 24,000 rpm, were smaller with a D 50% value of 1.41 mm and had a narrower size distribution, as indicated by the span value of 2.09. Microspheres produced at 9,500 and 13,000 rpm were also more uniform in size than $D_1$ with span values of 2.44 and 1.73, respectively.

The diltiazem HCI loaded PLA 109K microspheres prepared by Method C were analyzed by differential scanning calorimetry (DSC). The diltiazem HCI used was crystalline having a thermal event at 217°C. PLA 109K showed a thermal event at 65.6°C. DSC thermograms of the microspheres prepared by Method C showed no detectable crystalline diltiazem HCI. Physical mixtures containing 10% w/w and 5% w/w diltiazem HCI in PLA 109K had thermal events at 215.2°C, 65.5°C and 214.0°C, 63.9°C, respectively. A DSC scan of a 3% diltiazem in PLA 109K physical mixture showed a thermal event at 65.9°C only, indicating that 3-5% is the limit of detection for a diltiazem HCI/PLA 109K system.

X-ray diffraction patterns show the absence of crystalline diltiazem HCI in the systems prepared by Method C, indicating that diltiazem HCI is present in an amorphous or solubilized state.

EXAMPLE 4

Diltiazem HCl loaded PLA 109K microsphere systems were prepared by Method D, a method involving the evaporation of solvent from a double emulsion (w/o/w emulsion). The drug was initially dissolved in a 5% w/v gelatin solution to form the internal aqueous phase. The polymer/dichloromethane solution was added to the drug solution and emulsified using the Ultra Turrax at 24,000 rpm for one minute. The resulting w/o emulsion was then emulsified into the external aqueous, phase (buffered to pH 10.0) using the Ultra Turrax at 8,000 rpm for two minutes. Microspheres with a starting loading of 10% w/w ($D_5$) were produced and had an entrapment efficiency of 96.8%. The starting loading was subsequently increased to 20% w/w ($D_6$); the microspheres produced had an entrapment efficiency of 85.5%, finally, microspheres with a starting loading of 30% w/w ($D_7$) had an entrapment efficiency of 71.5%. As the starting loading was increased from 10% w/w to 30% w/w the entrapment efficiency decreased from 96.8% to 71.5%.

Using Method D, it was possible to prepare microspheres with up to 30% w/w starting loading, whereas with Method C it was not possible to exceed a starting loading of 10% w/w due to the limited solubility of the diltiazem HCl in the co-solvent (methanol). For Method D, in order to control the viscosity of the primary emulsion, the drug to gelatin ratio was kept at 0.1 g : 0.1 ml. Therefore, it was not possible to exceed a starting loading of 30% w/w as the diltiazem HCl necessary to achieve a drug loading of greater than 30% w/w would not dissolve in the gelatin solution.

Method D resulted in batches with lower percentage yield (54.0 - 63.5%) than those obtained using Method C (79.5 - 86.0%). This may be due to greater loss of product to vessel surfaces and homogenizer heads etc. during the two step process involved in Method D. Entrapment efficiency results shown in Table 2, for Methods C and D were comparable, ranging from 63.8-98.7% for Method C to 71.5-96.8% for Method D.

TABLE 2

| Lot No. | Starting Loading % w/w | Actual Loading % w/w | Entrapment efficiency % | Yield % | D 10% mm | D 50% mm | D 90% mm | Span |
|---|---|---|---|---|---|---|---|---|
| 5 | 5 10 | 9.68 | 96.8 | 60.0 | 1.37 | 5.39 | 15.81 | 2.65 |
| 6 | D20 | 17.10 | 85.5 | 54.0 | 3.34 | 15.95 | 42.97 | 2.60 |
| 7 | D30 | 21.46 | 71.5 | 63.5 | 2.88 | 9.43 | 60.65 | 4.39 |

SEM photomicrographs of the systems produced by Method D showed the particles to be spherical, however the surfaces of these particles showed some irregularities. System $D_7$, 30% w/w starting loading had some drug crystals present between the microspheres and on the microsphere surfaces. Particle size distribution plots for microsphere system $D_5$, showed bimodal distribution of particles whereas particle size distribution plots for microsphere system $D_7$ show multimodal distribution, which may be due to the presence of drug crystals, polymer debris or very small particles. Microsphere size was found to increase with increased drug loading. Microsphere system $D_5$ 10% w/w starting loading, had a D10% of 1.37 mm and a D90% of 15.81 mm compared to a D 10% of 2.99 mm and a D90% of 60.65 mm for ($D_5$), 30% w/w starting loading.

EXAMPLE 5

In-Vitro Release Studies: The effect of particle size on the release of diltiazem HCl from 10% loaded PLA 109K microspheres

Prior to carrying out *in vitro* release studies, the saturated solubility of diltiazem HCl was measured in order to establish sink conditions. Diltiazem HCl was found to have Cs values of 2.39 g/l in phosphate buffer pH 7.4, and greater than 1 g/ml in pH 1.2. The microsphere systems studied had a diltiazem HCl loading of 10% w/w and were prepared by Method C.

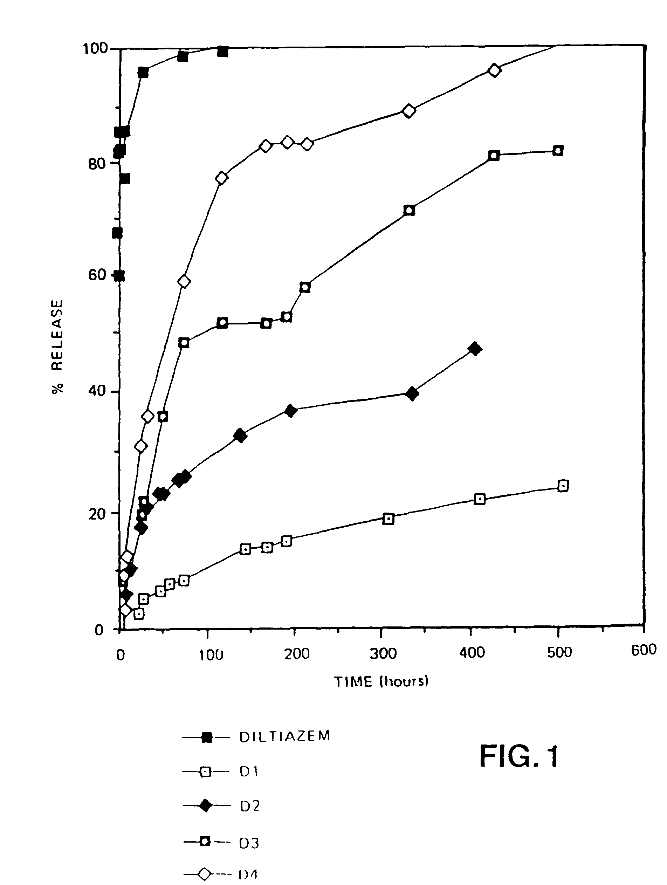

Release profiles of diltiazem HCl from PLA 109K microspheres obtained in isotonic phosphate buffer pH 7.4 showed release to be retarded when compared with release of pure diltiazem. HCl. Secondly, release was found to be related to the size of the particles. For a given mass of particles, the larger size microsphere released diltiazem at a slower rate than the smaller size particles. The release profiles are in rank order from the largest microspheres, $D_1$ produced at 8,000 rpm with a D50% of 12,56 mm, to $D_4$ produced at 24,000 rpm with a D50% of 1.41 mm (Fig. 1). Release profiles of diltiazem HCl from PLA 109 K microspheres obtained in SGF pH 1.2 also showed release to be retarded when compared with release of pure ditiazem HCl. In SGF, there was a high initial "burst" effect, smaller particles released up to 60% of drug in the first 24 hours. This was probably due to the solubility of the drug being greater in simulated gastric fluid (SGF) than in phosphate buffer. The release profiles of the larger microsphere systems $D_1$ (D50% of 12.56 mm, to $D_4$ produced at 24,000 rpm with a D50% of 1.41 $\mu$m.

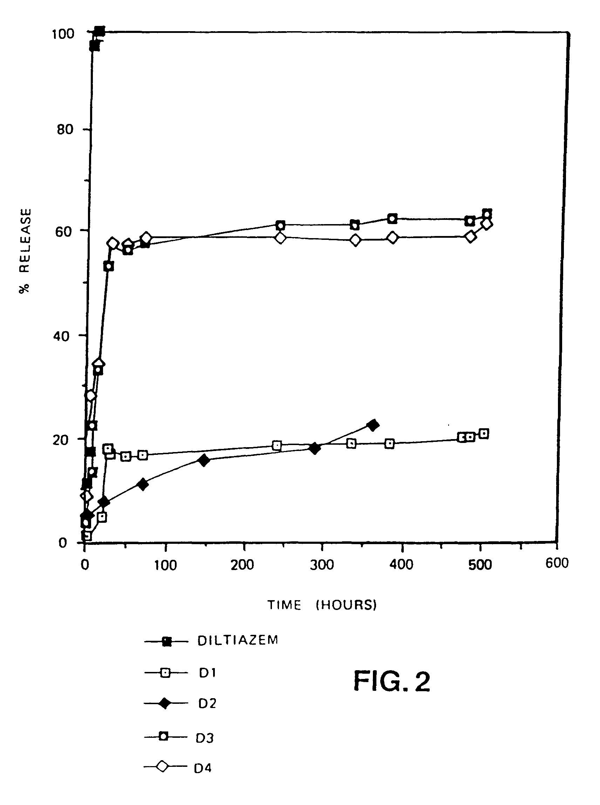

Release profiles of diltiazem HCl from PLA 109K microspheres obtained in SGF pH 1.2 also showed release to be retarded when compared with release of pure diltiazem HCl. In SGF, there was a high initial "burst" effect, smaller particles released up to 60% of drug in the first 24 hours. This was probably due to the solubility of the drug being greater in SGF than in phosphate buffer. The release profiles of the larger microsphere system $D_1$ (D50% of 12.56 mm) and $D_2$ (D50% of 5.76 mm) were similar: drug release from these systems was very slow. The smaller systems $D_3$ (D50% of 2.86 mm) and $D_4$ (D50% of 1.41 mm) also had similar release profiles and released diltiazem at a faster rate (Fig. 2).

Release profiles were fitted to Equation 2, which describes the release of drug from a spherical matrix. The rate constant K is defined by the Higuchi type equation. The best estimates of K by non-linear least square and values of correlation coefficient are given below in Table 3. The fits were considered relatively good in phosphate buffer pH 7.4, as measured by the correlation coefficients. Release appeared to be primarily diffusion controlled.

TABLE 3

| Lot No. | D 50% mm | K | correlation |
|---------|----------|---|-------------|
| $D_1$ | 12.56 | 0.00382 | 0.099714 |
| $D_2$ | 4.76 | 0.01008 | 0.97049 |
| $D_3$ | 2.86 | 0.01782 | 0.99170 |
| $D_4$ | 1.41 | 0.29023 | 0.99608 |

The correlation coefficients of the release data in pH 1.2 were poor. When the release data for the first five hours of diltiazem release in SGF was fitted to Equation 2, the correlation coefficients were much improved, indicating that release at the earlier time points was diffusion controlled. Values of K were found to be inversely proportional to particle size. As the D 50% decreased from 12.85 mm ($D_1$) to 1.41 mm ($D_4$), the value of K increased from 0.00382 to 0.29023 in phosphate buffer, and from 0.00783 to 0.03586 for release in the first five hours in SGF.

Release studies were stopped after 500 hours. The microspheres were collected, dried and assayed after dissolution in SGF. The drug content of the microspheres was as follows $D_1$:6.70% w/w; $D_2$: 7.42% w/w; $D_3$3.06% w/w; $D_4$: 2.35% w/w, i.e., the drug had not degraded in the microspheres. Subsequent release from these systems may have been controlled by degradation of the polymer.

EXAMPLE 6

Diltiazem base is soluble in dichloromethane, therefore Method A, a method involving the evaporation of solvent from and o/w emulsion, the oil phase being formed by dissolving the drug and polymer in dichloromethane was suitable for the preparation of diltiazem base loaded PLA 109K microspheres. All batches were prepared using a 1:11 ratio of PLA 109K dichloromethane. Microspheres with a starting loading of 10% w/w up to 80% w/w were prepared. The oil phase was homogenized into the external aqueous phase (buffered to pH 10.0) using the 1KA Ultra Turrax at 8,000 rpm for all systems except $Db_1$. As shown in Table 4, entrapment efficiencies in the range 61.85 - 88.5% were achieved. Product yield was high at all loadings, ranging from 83.0% to 97.0%.

TABLE 4

| Lot No. | Starting Loading % w/w | Actual Loading % w/w | Entrapment efficiency % | Yield % | D 10% mm | D 50% mm | D 90% mm | Span |
|---------|----------|----------|----------|---------|----------|----------|----------|------|
| $Db_1$ | 10 | 6.99 | 69.9 | 84.5 | 2.62 | 18.58 | 76.7 2 | 4.00 |
| $Db_2$ | 10 | 7.63 | 76.3 | 83.0 | 1.21 | 3.27 | 7.17 | 1.82 |
| $Db_3$ | 20 | 16.61 | 83.04 | 97.0 | 1.51 | 4.02 | 7.25 | 1.43 |
| $Db_4$ | 30 | 18.53 | 61.8 | 87.5 | 1.19 | 3.45 | 6.10 | 1.42 |
| $Db_5$ | 50 | 36.27 | 72.53 | 88.0 | 1.27 | 4.02 | 7.72 | 1.61 |
| $Db_6$ | 50 | 33.43 | 66.86 | 95.5 | 1.69 | 5.86 | 8.90 | 1.23 |
| $Db_7$ | 80 | 70.78 | 88.5 | 86.3 | 2.75 | 13.07 | 27.0 3 | 1.23 |

TABLE 4   (continued)

| Lot No. | Starting Loading % w/w | Actual Loading % w/w | Entrapment efficiency % | Yield % | D 10% mm | D 50% mm | D 90% mm | Span |
|---|---|---|---|---|---|---|---|---|
| $Db_8$ | 90 | not possible | - | - | - | - | - | - |

Using a 10% w/w starting loading, microsphere system $Db_2$ was prepared using the 1KA Ultra Turrax at the lowest speed setting of 8,000 rpm to emulsify the oil phase into the external equeous phase. Microspheres with a D50% of 3.27 mm were produced (diltiazem HCl loaded microspheres prepared at the same homogenization speed using Method C had a larger D50% of 12.56 mm).

Microsphere system $Db_1$ was prepared using a T25 stirrer at a much lower speed setting of 1,400 rpm to emulsify the oil phase into the external aqueous phase. The microspheres were larger as expected with a D50% of 18.58 mm. The microspheres prepared with the IKA Ultra Turrax had a higher entrapment efficiency than those prepared with the T25 stirrer. Since it was established that the IKA Ultra Turrax could efficiently produce small particles at 8,000 rpm using Method A, this speed setting was used for subsequent batches $Db_3$ -$Db_7$.

Diltiazem base is less soluble in water than diltiazem HCl, therefore significantly higher drug loading could be achieved (up to 80% w/w starting loading) as there was very little loss of drug to the external aqueous phase. It was not possible to exceed a starting loading of 80% w/w as the amount of diltiazem base necessary to achieve greater loading would not dissolve in the given amount of dichloromethane. Using Method A with the Ultra Turrax, microspheres with entrapment efficiencies in the range 61.8-88.5% were produced. Entrapment efficiency was independent of starting loading. Product yield ranged from 83.0% to 97.0% and was also independent of starting loading.

Diltiazem base loaded PLA 109K microsphere systems prepared by Method A at drug loadings of 10, 20, 50 and 80% w/w were analyzed by DSC. The diltiazem base starting material used had a thermal event at 105°C. PLA 109K had a thermal event at 65.6°C. Physical mixtures containing 10% and 30% w/w diltiazem base had thermal events at 64.8°C, 104.4°C, 64.6°C and 104.7°C, respectively. The limit of detection for a diltiazem base/PLA 109K system is 10% w/w. DSC thermograms of systems $Db_2$, $Db_3$ and $Db_5$ showed no detectable crystalline diltiazem base, whereas $Db_7$ had a thermal event at 105.4°C, indicating the presence of crystalline diltiazem base.

SEM photomicrographs of diltiazem base loaded PLA 109K systems prepared by Method A using the Ultra Turrax showed spherical particles. The surfaces of the particles were smooth. However, at 50% w/w loading there was some debris on the microsphere surfaces and in between the microspheres; this may be polymer or drug crystals. At 80% w/w loading there was a considerable amount of drug crystals present.

With the exception of $Db_7$, particle size results of all microsphere systems prepared with the Ultra Turrax were very reproducible, with D10% ranging from 1.19-1.69 mm, D50% ranging from 3.27-5.86 mm and D90% ranging from 6.10-8.90 mm. Microspheres of system $Db_7$ were larger with a D 50% of 13.07 mm. Microsphere systems prepared with the Ultra Turrax ($Db_2$ - $Db_6$) were also reasonably uniform in size with span values ranging from 1.23-1.82. In contrast, microspheres of microsphere system $Db_1$, prepared at 1,400 rpm with the T25 stirrer, were much larger (D50% of 18.58 mm) and had a wider size distribution, as indicated by a span value of 4.00 and particle size distribution plots.

EXAMPLE 7

**In-vitro release studies: The effect of drug loading on the release of diltiazem base from PLA 109K microspheres**

Prior to carrying out *in vitro* release studies, the saturated solubility of diltiazem base was measured in order to establish sink conditions. Diltiazem base was found to have Cs values of 227.47 g/l in SGF pH 1.2 and 1.06 g/l in phosphate buffer pH 7.4. The microsphere systems studied had diltiazem base starting loadings of 10% w/w, 20% w/w, 30% w/w, and 50% w/w, D 50% of 3.27 mm, 3.45 mm, 4.02 mm, respectively, and were prepared by Method A using the Ultra Turrax.

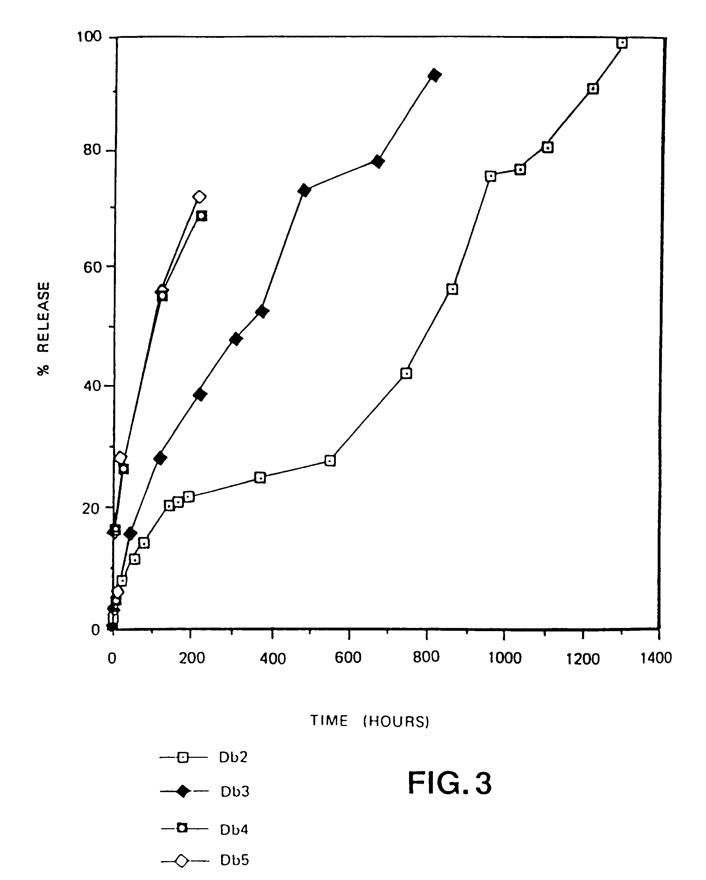

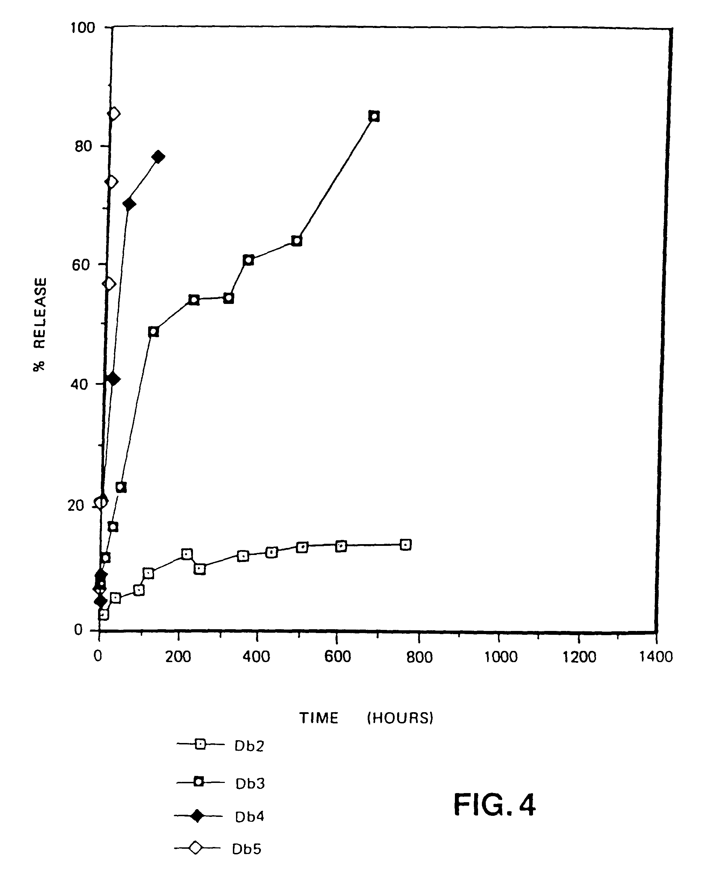

Release profiles of diltiazem base from PLA 109K microspheres in isotonic phosphate buffer pH 7.4 showed release to be dependent on percentage drug loading. Microspheres at the lower drug loading of 10% w/w released diltiazem base at a slower rate. The release profiles were in rank order the lowest drug loading 10% w/w to the highest drug loading 50% w/w. There was very little difference in the release profiles of microspheres at 30% and 50% w/w (Fig. 3). Release profiles of diltiazem base from PLA 109K microspheres in SGF pH 1.2 also showed release to be dependent on percentage drug loading. The release profiles were again in rank order (Fig. 4). There was a notable difference between the release profiles of microspheres at 30% and 50% w/w loading. Microspheres at 50% w/w loading released diltiazem base at a very fast rate. Diltiazem base had a high solubility in SFG. Additionally, at high drug loadings there may be drug at or near to the surface of the microspheres; this may explain the very fast release rate from microspheres at 50% w/w loading. The release profiles in phosphate buffer and in SFG were fitted to Equation 2.

The fits may be considered relatively good, as measured by the correlation coefficients. Release in phosphate buffer appeared to be primarily diffusion controlled. Release in SGF from microspheres with high drug loading also appeared to be primarily diffusion controlled. At lower drug loadings (20% w/w), release was diffusion controlled at the earlier time points. Later release may have been controlled by a combination of diffusion and polymer degradation as indicated by SEM photomicrographs after dissolution. Values of K were found to be proportional to A, the mass of drug initially present. As the starting drug loading increased from 10% w/w ($Db_2$) to 50% w/w ($Db_5$), the value of K increased from 0.00378 to 0.02130 in phosphate buffer and from 0.00229 to 0.22015 in SGF.

SEM phomicrographs taken after dissolution of microsphere system $Db_3$, 20% w/w drug loading, showed complete degradation of the microspheres in both phosphate buffer and SGF suggesting that release at later time points was controlled by a combination of diffusion of the drug into the dissolution medium and degradation of the polymer. Microspheres at 50% w/w drug loading, $Db_5$ remained intact in SGF, suggesting that at higher drug loading release in SGF was totally diffusion dependent due to the high solubility of the drug in SGF. These microspheres were degraded in phosphate buffer as release in phosphate buffer was slower due to the reduced solubility of the drug and the faster degradation of the polymer at pH 7.4.

EXAMPLE 8

Diltiazem base loaded PLA 109K microspheres were prepared by Method A using the Microfluidiser (Trade Mark), high pressure homogenizer, to homogenize the oil phase into the external aqueous phase (buffered to pH 10.0). The Microfluidiser was operated at 10,000 psi for one cycle. All batches were prepared using a 1.11 ratio of PLA 109K : dichloromethane. As shown in Table 5, microspheres with a starting loading of 10% w/w were prepared and an entrapment efficiency of 53.7% was achieved. Subsequent systems with starting loadings of 20%, 30% and 50% w/w were prepared. Entrapment efficiencies of 68.1%, 65.2% and 84.5% respectively were achieved and product yields in the range of 56.0% to 67.5% were obtained.

TABLE 5

| Lot No. | Starting loading % w/w | Actual loading % w/w | Entrapment efficiency % | % yield | Z average nm |
|---|---|---|---|---|---|
| $Db_9$ | 10 | 5.37 | 53.7 | 67.0 | 302.5 |
| $Db_{10}$ | 20 | 13.61 | 68.1 | 60.0 | 294.0 |
| $Db_{11}$ | 30 | 19.56 | 65.2 | 67.5 | 307.5 |
| $Db_{12}$ | 50 | 42.08 | 84.2 | 56.0 | 310.0 |

Whether the Ultra Turrax or the Microfluidiser was used for homogenization, the range of entrapment efficiency was not affected; the Ultra Turrax produced batches with entrapment efficiencies in the range of 61.8-88.5% while the Microfluidiser produced batches with entrapment efficiencies in the range of 53.7-84.2%. However, the percentage yields were lower when the Microfluidiser was used for homogenization. That is, yields of 56.0-67.0% were achieved with the Microfluidiser compared to 83.0-97.0% with the Ultra Turrax. This may be due to the difficulty in fully recovering product from the Microfluidiser.

Microsphere systems prepared by Method A using the Microfluidiser, $Db_9$-$Db_{12}$, had Z average mean size values in the range of 294-310 nm, illustrating that the Microfluidiser is a very effective homogenizer capable of producing very fine emulsion droplets. The particle size was independent of drug loading. Systems with a 10% w/w loading had a Z average mean size value of 302.5 nm while systems with 50% w/w loading had a Z average mean size value of 310.0 nm. The particles produced by this method had smooth surfaces and were spherical in shape. These systems were also uniform in size as seen in SEM photomicrographs of the systems.

EXAMPLE 9

**The effect of drug loading and pH on the *in vitro* release of diltiazem base from PLA 109 K microspheres prepared with the Microfluidiser**

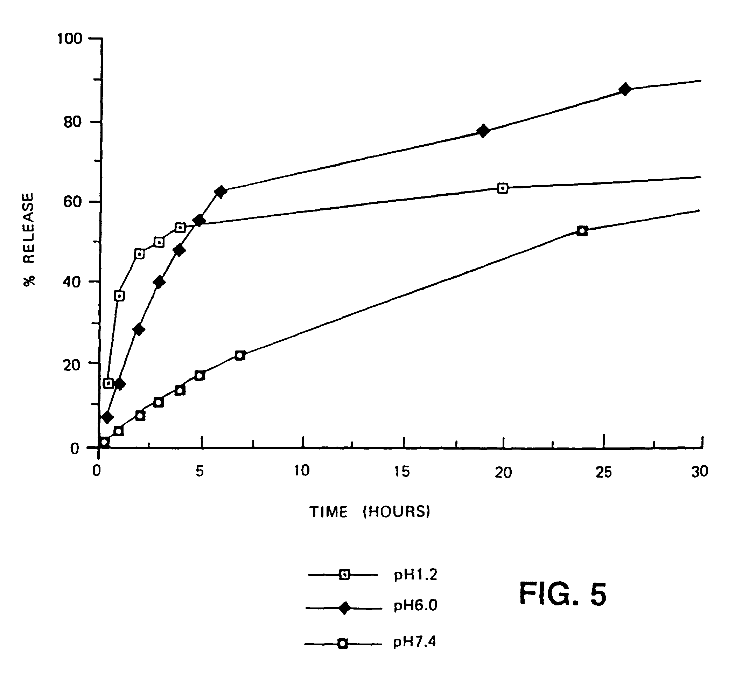

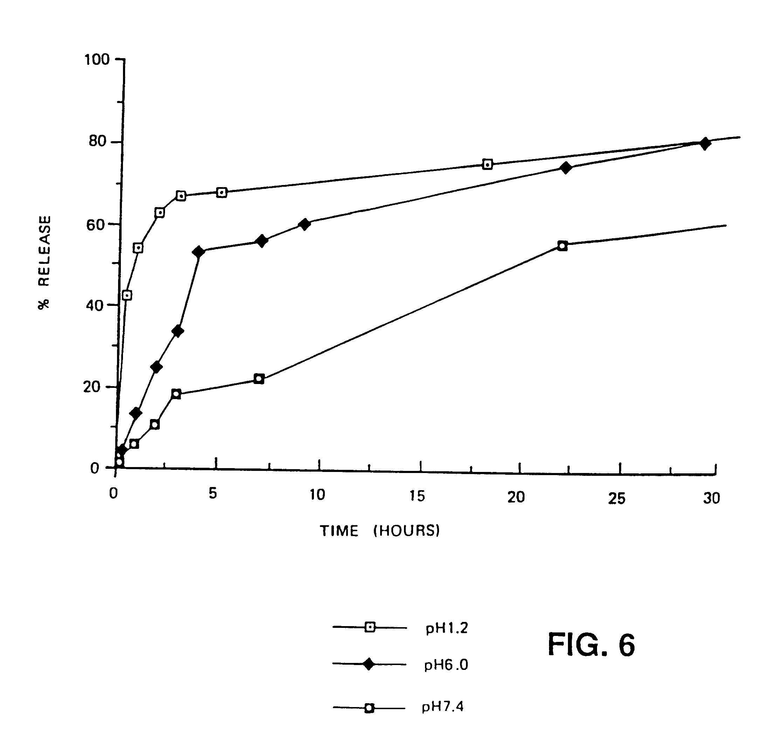

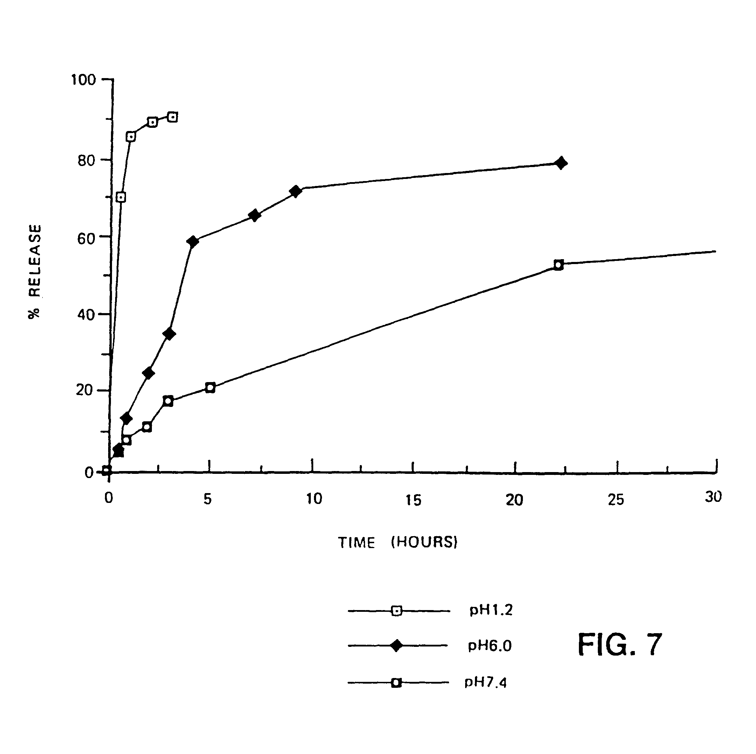

The microsphere systems studied had diltiazem base starting loadings of 10% w/w, 20% w/w, 30% w/w and 50% w/w, Z average mean sizes of diameter (D50%) 302.5 nm, 294.0 nm, 307.5 nm, and 310.0 nm, respectively, and were prepared by Method A using the Microfluidiser. Release profiles of diltiazem base from PLA 109K microspheres, obtained in isotonic phosphate buffer pH 7.4, showed release to be dependent on drug loading at the earlier time points (i.e., from 1-25 hours). Release profiles were superimposable over the 200 hour study period, the effect of drug loading on release was not seen. In contrast, release of diltiazem base from PLA 109K microspheres in SGF was found to be dependent on drug loading. Release profiles were in rank order, microspheres with 10% w/w starting loading released diltiazem base at the slowest rate, microspheres with 50% w/w starting loading released diltiazem base almost immediately. Figs. 5-7 show release from 20%, 30% and 50%, respectively, diltiazem base loaded PLA 109K nanospheres at pH 1.2, pH 6.0 and pH 7.4 (prepared according to Example 8). Because of the delivery characteristics of these microcapsules over a 24 hour period, these formulations are appropriate for once-daily administration of diltiazem.

In both media, release of diltiazem base from microsphere systems prepared with the Microfluidiser was much faster when compared to release from microsphere systems prepared with the Ultra Turrax. At 168 hours, $Db_9$ microspheres (10% w/w loading, prepared with the Microfluidiser) had release 100% diltiazem base in phosphate buffer pH 7.4 compared to only 16.06% from $Db_2$ (10% w/w loading, prepared with the Ultra Turrax).

Release profiles were fitted to Equation 2. The best estimates of K by non-linear least squares and values of correlation coefficient are given below. The fits may be considered relatively good as measured by the correlation coefficients. Release appeared to be diffusion controlled.

TABLE 6:

| The best estimates of K by non-linear least squares and values of correlation coefficient for diltiazem base release in phosphate buffer pH 7.4 | | | |
|---|---|---|---|
| Lot No. | Drug loading % w/w | K | correlation |
| $Db_9$ | 5.37 | 0.03572 | 0.98990 |
| $Db_{10}$ | 13.61 | 0.04064 | 0.99201 |
| $Db_{11}$ | 19.56 | 0.04234 | 0.99107 |
| $Db_{12}$ | 42.08 | 0.04358 | 0.99816 |

The correlation coefficients of the release data in SGF pH 1.2 were poor when plotted over the entire course of the release study, particularly at the lower drug loading of 10% and 20% w/w. The fits were considered relatively good for diltiazem release at the higher drug loading of 50% w/w, indicating that release was diffusion controlled. When the release data for the first five hours of diltiazem release in SGF was fitted to Equation 2, the correlation coefficients were much improved, indicating that release at the earlier time points was diffusion controlled and release was subsequently controlled by degradation of the polymer. This may be due to the fact that at the lower drug loadings there was less drug at or near the surface of the microspheres; therefore, there was less drug accessible to the dissolution medium. Polymer degradation must have occurred in order to allow drug release. Values of K were found to be proportional to drug loading. As the loading increased from 5.37% w/w ($Db_9$) to 42.08% w/w ($Db_{12}$), the value of K increased from 0.03572 to 0.04358 in phosphate buffer and from 0.07678 to 0.44293 for release in the first five hours in SGF.

TABLE 7:

| The best estimates of K by non-linear least square and values of correlation coefficient for diltiazem base release in SGF pH 1.2 | | | | | |
|---|---|---|---|---|---|
| Lot No. | Drug loading % w/w | Release in SGF - Hours 0-200 | | Release in SGF - Hours 1-5 | |
| | | K | correlation | K | correlation |
| $Db_9$ | 5.37 | 0.03620 | 0.82297 | 0.07678 | 0.98313 |
| $Db_{10}$ | 13.61 | 0.08651 | 0.90619 | 0.12027 | 0.94174 |
| $Db_{11}$ | 19.56 | 0.17499 | 0.93772 | 0.18784 | 0.95852 |
| $Db_{12}$ | 42.08 | 0.44293 | 0.96204 | 0.44293 | 0.96204 |

EXAMPLE 10

**Diltiazem *in-vivo* studies**

Diltiazem nanoparticles were prepared in accordance with Examples 8 and 9 at starting drug loading of 20%, 30% and 50% for enclosure into a capsule (e.g., gel capsule). Diltiazem HCl (USP Grade) was used as the active ingredient. Poly-D, L-lactide (R206 Boehringer Ingelheim), molecular weight 109,000, inherent viscosity of 1.0, was used as the biodegradable polymer. The potency of the capsules was determined using high performance liquid chromatography in accordance with the method detailed in the USP.

Release characteristics of the test product were established prior to the study in accordance with the Paddle Method of U.S. Pharmacopoeia XX at 37∞C and 75 rpm. The test product was shown to exhibit the following *in vitro* release profile:

| Time (hr) | % release |
|-----------|-----------|
| 2 | 10-30 |
| 4 | 30-60 |
| 8 | 60-80 |
| 24 | ≥80 |

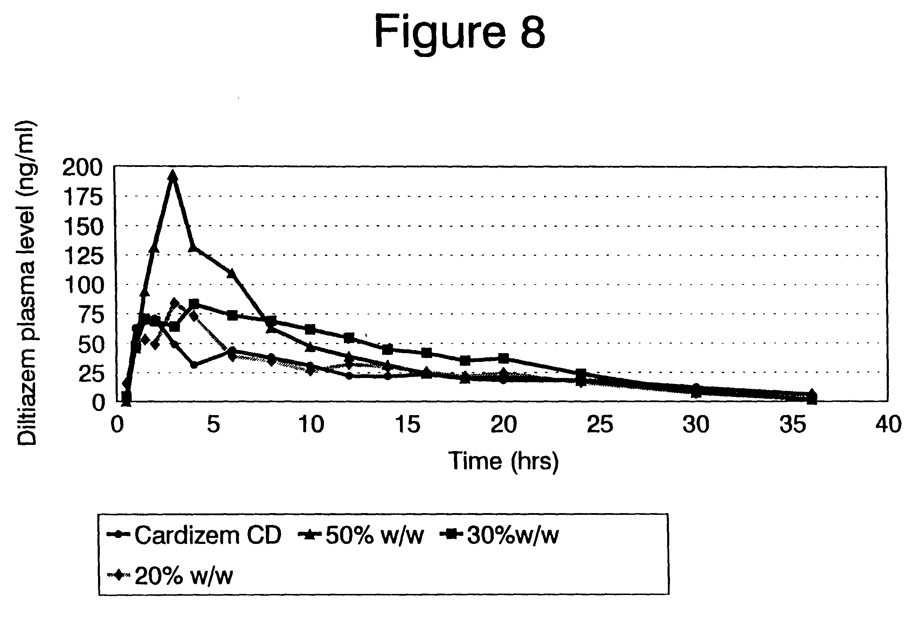

180 mg of the three test products were administered to human volunteers. Cardizem CD 180 mg (Trade Mark, Marion Merrell Dow) was used as the reference in this study. Microfluidised microcapsules/nanoparticles, as prepared in accordance with Example 8, were used in preference, since the Z average mean diameter ranges (294-310 nm) and starting loading (10% to 50% w/w) provide characteristics which are most preferable for use in these preparations. The diltiazem plasma levels were measured over a period of 36 hours as shown in Fig. 8. Both the 20% and 30% starting loading preparations showed desirable controlled release and bioavailability characteristics over at least a 24 hour period of time.

EXAMPLE 11

**Captopril *in vivo* Studies**

Captopril nanoparticle capsules are prepared using s spray drying technique. Captopril (USP grade) is used as the active ingredient. Poly-D,L- lactide (R203 Boehringer Ingelheim), molecular weight 16,000 is used as the biodegradable polymer.

The potency of the test product is determined by HPLC by the manner outlined in USP. Release characteristics of the test product are established prior to commencement'of the study, using the *in vitro* test procedure described in Example 10. The test product is shown to exhibit the following *in vitro* release:

| Time (hr) | % release |
|-----------|-----------|
| 1 | 10-40 |
| 4 | 20-60 |
| 8 | 40-80 |
| 16 | >80 |

2 x 37.5 mg captopril containing amounts of the formulation are administered to volunteers (humans). Capoten (Trade Mark) (Squibb), is used as the reference in this study. Captopril microfluidised microcapsules/nanoparticles, as prepared in accordance with Example 8, as indicated above, are suitable for a once-daily preparation.

EXAMPLE 12

***In-vitro* release from Hydromorphone loaded microcapsules**

Hydromorphone microspheres were prepared using a double emulsion technique as follows:

EP 0 754 034 B1

TABLE 8

| | Lot No. | |
|---|---|---|
| | Hm32 | Hm30 |
| R206 (poly-D,L-lactide; mw 109,000) | 3.0 g | 3.0 g |
| Hydromorphone HCl | 0.3 g | 0.3 g |
| Methylene Chloride | 6.0 ml | 8.0 ml |
| Gold gelatin (pharmaceutical grade) | 1.0 ml | 1.0 ml |
| Tris buffer (0.27% PVA; pH 9) | 50.0 ml | 50.0 ml |
| Acetone | ---- | 3 ml |
| Actual Hydromorphone loading | 4.80% | 3.06% |
| Average size | | |

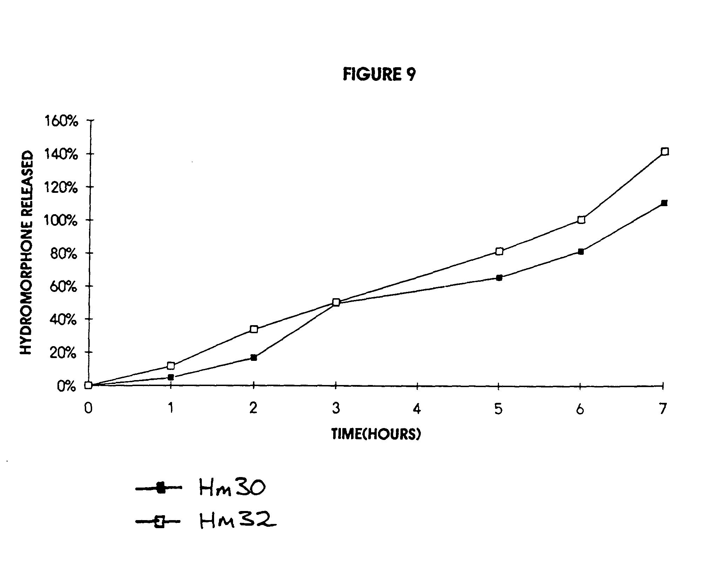

R206 polymer was dissolved in the solvent methylene chloride and hydromorphone was dissolved in the gelatin. The drug/gelatin mixture was emulsified into the polymer/solvent (S10 homogenizing shaft size/13,500 for 2_ min for Hm32 and 2 min for Hm30). The resulting emulsion was itself emulsified into the aqueous Tris buffer (S25 homogenizing shaft size/13500 for 3_ min for Hm32 and 3 min for Hm30) to form microcapsules. The Hm32 microcapsules were stirred 1,000 rpm for 1_ hours to harden the microcapsules. The Hm30 microcapsules were stirred at 1,200 rpm for 15 minutes, the acetone was added and the mixture again stirred at 1,200 rpm for an additional 1_ hours. The microcapsules were recovered by centrifuging at 13,500 rpm for 15 minutes and drying under a vacuum. The microcapsules were from 20-30 μm in diameter. Fig. 9 shows the dissolution of hydromorphone from these hydromorphone microspheres encased in gelatin capsules in simulated gastric fluid (0.1 M HCl).

EXAMPLE 13

**In-vitro release from Nifedipine loaded microcapsules**

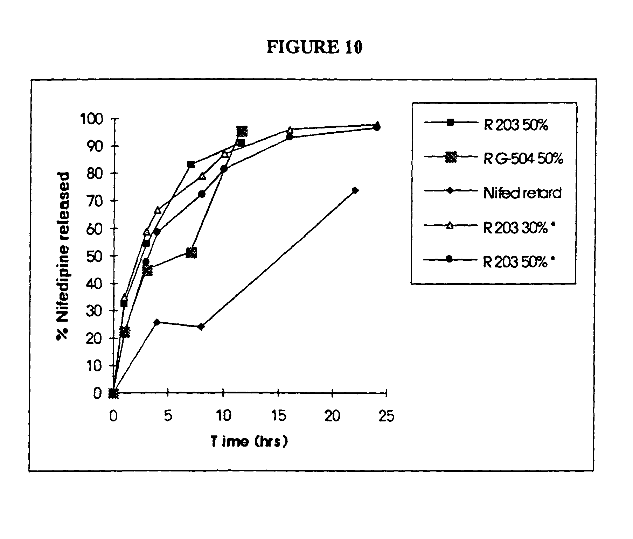

Nifedipine loaded microspheres, with loadings up to 50% w/w drug loading using either R203 (poly-D,L-lactide; 16000 mw) or RG 504 (poly-D,L-lactide-co-glycolide; 50:50) were prepared using a procedure similar to that of Example 8 for diltiazem base loaded microspheres. The microspheres were < 5 μm in diameter with entrapment efficiencies greater than 90% in all cases. As shown in Fig. 10, the release under sink conditions of nifedipine from 50% loaded R203 and 50% loaded RG 504 microspheres are compared to the release of nifedipine from 30% loaded R203 and 50% loaded RG 504 microspheres that were mixed with lactose and tabletted prior to dissolution runs (Nifed retard, Rowa Pharmaceutical, as a control) over a period of 25 hours.

EXAMPLE 14

**Effervescent Formulations**

The following diltiazem effervescent formulations were prepared from the microcapsule preparations of this invention similar to Example 8:

TABLE 9

| | Lot No. | | | | | |
|---|---|---|---|---|---|---|
| wt% of final product | EFF6 | EFF9 | EFF10 | EFF16 | EFF19 | EFF24 |
| Diltiazem microspheres | 5.0 (20% loading) | 10.0 (20% loading) | 10.0 (20% loading) | 5.0 (20% loading) | 12.0 (50% loading) | 12.0 (20% loading) |
| Sodium bicarbonate | 40.0 | 40.0 | 40.0 | 20.0 | 40 | 40 |

TABLE 9 (continued)

| wt% of final product | EFF6 | EFF9 | EFF10 | EFF16 | EFF19 | EFF24 |
|---|---|---|---|---|---|---|
| | | | Lot No. | | | |
| Potassium bicarbonate | --- | --- | --- | 22.5 | --- | --- |
| Citric acid anhydrous | 25.0 | 17.0 | 20.0 | 28.5 | 25.0 | 25.0 |
| Sorbitol | 10.0 | --- | | 18.0 | --- | --- |
| Aspartame | 10.0 | 10.0 | 10.0 | 5.0 | 10.0 | 10.0 |
| Explotab | 10.0 | 10.0 | 17.0 | --- | 5.0 | --- |
| Kollidon 30 | --- | 10.0 | --- | --- | --- | --- |
| Starch | --- | --- | --- | --- | 5.0 | --- |
| Methocel | --- | --- | --- | --- | --- | 5.0 |
| Avicel pH 101 | --- | --- | --- | --- | --- | 8.0 |
| Flavour | --- | 3.0 | 3.0 | 3.0 | 3.0 | |
| Tablet wt | 1.0 g | 1.0 g | 1.0 g | 1.0 g | 1.0 g | 1.0g |

The microspheres of formulation EFF 6 were initially coated by spraying the microcapsules with a 1% solution of sodium docusate. The coated microspheres were dried in a vacuum oven overnight and dry blended with the other listed excipients and tabletted.

The ingredients of formulation EFF 9, EFF 16 and EFF 19 were dry-blended together and tabletted.

The microspheres of EFF 10 were blended with the acid and base phases and a wet granulation was prepared using a 1% solution of sodium docusate. The granulate was dried in the vacuum oven overnight and ground to a powder. The granulate was blended with the remaining dry excipients and tabletted.

A wet granulate was prepared from the microspheres, Methocel and the acid and base components using a 5% heated sodium docusate solution as the granulating agent in EFF 24. The granulate was dried under vacuum and ground to powder form The granulate was blended with the remaining dry excipients and tabletted.

These effervescent formulations contain diltiazem base loaded microcapsules similar to those given in Example 8, whose general release properties in various pH environments are given in Figs. 5-7 and accompanying text and whose bioavailability results are outlined in Fig. 8 and accompanying text. As shown in Figs. 5-7 (e.g., release profiles at pH 7.4 and pH 1.2), the aqueous solubility of the diltiazem increases at pHs below its $pK_a$ (around 8), with an appreciable increase in solubility and release observed at pHs < 7. Thus, effervescent formulations containing diltiazem base desirably are adjusted to have a suspension pH of 7 or above to prevent the burst release of diltiazem in the suspension or in the subject prior to reaching the much more acidic environment of the stomach. By controlling the pH of the suspended effervescent formulation, the release of diltiazem will be minimized until the diltiazem is delivered to absorption sites for diltiazem (e.g., stomach, small intestine). In this way, delivery of diltiazem can be optimized while still maintaining a desirable controlled, sustained release with a formulation that has the advantages inherent in a effervescent dosage form.

The pH of effervescent formulations can be manipulated from about pH 5 to pH 8 by, for instance, increasing the relative amount of sodium bicarbonate (to raise the pH) or increasing the citric acid component (to lower the pH). For instance, the diltiazem formulations of Table 9 can be adjusted to around pH 7.4 to minimize the burst effect of the microcapsules in the effervescent drink solution.

Similar effervescent formulations can also be formulated at appropriate pHs for nifedipine, captopril, hydromorphone etc. to advantageously optimize their bioavailability when administered orally to the subject. For instance, because nifedipine is very poorly water soluble with its solubility increasing only at pHs< 2-3, incorporation of nifedipine loaded microcapsules into an effervescent matrix should not negatively affect its release properties. That is, negligible drug will be released in the effervescent drink (burst effect) prior to oral intake and thus the majority of the nifedipine will be delivered in a controlled manner to the site(s) of its absorption. On the other hand, hydromorphone has a high aqueous solubility around effervescent solutions of around neutral pH and, therefore, release will have started to a certain extent prior to intake. Alternatively, the pH of the hydromorphone effervescent formulation can be raised to about pH 8 to reduce the burst effect or the formulation's pH can be lowered below approximately pH 6 and the microcapsules can be coated or sprayed with an enteric coating to prevent drug release until after they pass through the stomach.

Effervescent formulations containing more than one type of drug loaded microcapsules are also envisioned by this invention. That is, microcapsules containing the same drug, for instance faster and slower releasing diltiazem microcapsules, can be combined in one effervescent formulation to improve the controlled sustained release profile for diltiazem. Alternatively, microcapsules containing different drugs, such as a calcium channel blocker (e.g., diltiazem, nifedipine) and a narcotic analgesic (e.g., hydromorphone) can be formulated in one effervescent formulation to simultaneously provide optimized delivery of both drugs in the patient friendly effervescent dosage form. That is, using diltiazem/hydromorphone as an example, microcapsules with the desired release profile for diltiazem (e.g., *in vitro* release in simulated gastric fluid) and formulated with hydromorphone loaded microcapsules in an effervescent formulation having a pH near or above pH 7 such as at pH 7.4. By selecting this relatively high pH for an effervescent drink solution, the delivery of diltiazem can be optimized without affecting the delivery profile of hydromorphone negatively.

**Claims**

1. An effervescent pharmaceutical formulation for the sustained and controlled oral administration of a pharmaceutically effective amount of a drug selected from a calcium channel blocker, an ACE inhibitor, a narcotic analgesic or analogues or combinations thereof, said formulation comprising microcapsules having a D 50 % between 100 nm and 900 nm in which the drug is entrapped in a biodegradable polymer and in which the pH of the formulation is adjusted to optimize delivery of the drug, wherein the formulation is adapted to disperse upon addition of water to form an effervescent drink.

2. An effervescent formulation according to Claim 1, wherein the drug is diltiazem or a combination of diltiazem and a narcotic analgesic and wherein the pH of the formulation is greater than 7.

3. An effervescent pharmaceutical formulation according to Claim 1 or 2, wherein the formulation is used to deliver the drug to a patient on a once-daily basis so as to achieve a therapeutic effect over a substantially 24 hour period.

4. An effervescent formulation according to Claim 1, wherein the drug is hydromorphone or a combination of hydromorphone and a calcium channel blocker and wherein the pH of the formulation is less than pH 6 or greater than pH 7.

5. An effervescent pharmaceutical formulation according to any preceding claim, wherein the drug loading of the microcapsules ranges from about 10% to 70% by weight.

6. An effervescent pharmaceutical formulation according to any preceding claim, wherein the microcapsules have a D 50% between about 200 nm and 400 nm.

7. An effervescent pharmaceutical formulation according to any preceding claim, wherein the drug loading of the microcapsules ranges from about 20% to 50% by weight.

8. An effervescent pharmaceutical formulation according to any preceding claim, wherein the drug is selected from diltiazem, verapamil, nifedipine, nimodipine, nicardipine, hydromorphone, codeine sulfate, oxycodone, dihydrocodeine tartrate, oxycodeinone, morphine, fentanyl, sufentanil, oxymorphone, buprenorphine, captopril, enalapril, lisonopril and mixtures thereof.

9. An effervescent pharmaceutical formulation according to Claim 8, wherein the drug is a mixture of nifedipine and hydromorphone.

10. A pharmaceutical formulation according to any preceding claim, wherein the polymer matrix comprises polylactide; polyglycolide; poly(lactic acid-co-glycolic acid); poly(e-caprolactone); poly(hydroxybutyric acid); polyortho-esters; polyacetals; polydihydropyrans; polycyanoacrylates; polypeptides; cross-linked polypeptides; and stereoisomers, racemic mixtures, co-polymers and polymer mixtures thereof.

11. A pharmaceutical formulation according to Claim 10, wherein the polymer matrix comprises poly-D,L-lactide.

**12.** A pharmaceutical formulation according to any preceding claim, wherein the release profile measured in accordance with the Paddle Method of U.S. Pharmacopoeia XX at 37°C and 75 rpm for the or each drug is substantially as follows:

    a) 10-30% release within 2 hours after administration;

    b) 30-60% release within 4 hours after administration;

    c) 60-80% release within 8 hours after administration; and

    d) ≥ 80% release within 24 hours after administration.

**13.** A pharmaceutical formulation according to any one of Claims 1-11, wherein the release profile measured in accordance with the Paddle Method of U.S. Pharmacopoeia XX at 37°C and 75 rpm for the or each drug is substantially as follows:

    a) 10-40% release within 1 hour after administration;

    b) 20-60% release within 4 hours after administration;

    c) 40-80% release within 8 hours after administration; and

    d) ≥ 80% release within 16 hours after administration.

**14.** A method for the manufacture of microcapsules according to any one of Claims 1-13, which comprises the steps of:

    a) dissolving or dispersing a drug and a biodegradable polymer in a solvent to form a mixture;

    b) microfluidising said mixture into an external phase to form an emulsion in which the emulsion droplets have a mean diameter less than 1 mm; and

    c) stirring said emulsion to form microcapsules having a size (D 50%) between about 100 nm and 900 nm.

**Patentansprüche**

**1.** Pharmazeutische Brauseformulierung zur lang anhaltenden und kontrollierten oralen Verabreichung einer pharmazeutisch wirksamen Menge eines Arzneimittels, ausgewählt aus einem Calciumkanalblocker, einem ACE-Inhibitor, einem Narkoanalgetikum oder Analogen oder Kombinationen hiervon, wobei die Formulierung Mikrokapseln mit einer D 50 % zwischen 100 nm und 900 nm enthält, in denen das Arzneimittel in einem biologisch abbaubaren Polymeren eingeschlossen ist und in denen der pH-Wert der Formulierung zur optimalen Abgabe des Arzneimittels eingestellt ist, wobei die Formulierung bei Zugabe von Wasser zur Dispersion unter Bildung eines sprudelnden Getränkes ausgelegt ist.

**2.** Brauseformulierung nach Anspruch 1, wobei das Arzneimittel Diltiazem oder eine Kombination von Diltiazem und einem Narkoanalgetikum ist und wobei der pH-Wert der Formulierung größer ist als 7.

**3.** Pharmazeutische Brauseformulierung nach Anspruch 1 oder 2, wobei die Formulierung zur einmal täglichen Abgabe des Arzneimittels an einen Patienten unter Erzielung einer therapeutischen Wirkung über einen Zeitraum von im wesentlichen 24 h eingesetzt wird.

**4.** Brauseformulierung nach Anspruch 1, wobei das Arzneimittel Hydromorphon oder eine Kombination von Hydromorphon und einem Calcium-kanalblocker ist und wobei der pH-Wert der Formulierung kleiner als 6 oder größer als 7 ist.

**5.** Pharmazeutische Brauseformulierung nach einem vorhergehenden Anspruch, wobei die Arzneimittelkonzentration der Mikrokapseln von etwa 10 bis 70 Gew.-% reicht.

6. Pharmazeutische Brauseformulierung nach einem vorhergehenden Anspruch, wobei die Mikrokapseln eine D 50 % zwischen etwa 200 nm und 400 nm aufweisen.

7. Pharmazeutische Brauseformulierung nach einem vorhergehenden Anspruch, wobei die Arzneimittelkonzentration der Mikrokapseln von etwa 20 bis 50 Gew.-% reicht.

8. Pharmazeutische Brauseformulierung nach einem vorhergehenden Anspruch, wobei das Arzneimittel ausgewählt ist aus Diltiazem, Verapamil, Nifedipin, Nimodipin, Nicardipin, Hydromorphon, Codeinsulfat, Oxycodon, Dihydrocodeintartrat, Oxycodeinon, Morphin, Fentanyl, Sufentanil, Oxymorphon, Buprenorphin, Captopril, Enalapril, Lisonopril und Gemischen hiervon.

9. Pharmazeutische Brauseformulierung nach Anspruch 8, wobei das Arzneimittel ein Gemisch von Nifedipin und Hydromorphon ist.

10. Pharmazeutische Formulierung nach einem vorhergehenden Anspruch, wobei die Polymermatrix Polylactid; Polyglycolid; Poly(milchsäurecoglycolsäure); Poly($\varepsilon$-caprolacton); Poly(hydroxybuttersäure); Polyorthoester; Polyacetale; Polydihydropyrane; Polycyanoacrylate; Polypeptide; vernetzte Polypeptide; und Stereoisomere, racemische Gemische, Copolymere und Polymergemische hiervon umfaßt.

11. Pharmazeutische Formulierung nach Anspruch 10, wobei die Polymermatrix Poly-D,L-lactid enthält.

12. Pharmazeutische Formulierung nach einem vorhergehenden Anspruch, wobei das Freisetzungsprofil, gemessen nach der Paddle-Methode der U.S.-Pharmacopoe XX bei 37 °C und 75 U/min, für das oder jedes Arzneimittel im wesentlichen wie folgt ist:

   a) 10 - 30 % Freisetzung innerhalb 2 h nach Verabreichung;
   b) 30 - 60 % Freisetzung innerhalb 4 h nach Verabreichung;
   c) 60 - 80 % Freisetzung innerhalb 8 h nach Verabreichung; und
   d) ≥ 80 % Freisetzung innerhalb 24 h nach Verabreichung.

13. Pharmazeutische Formulierung nach einem der Ansprüche 1-11, wobei das Freisetzungsprofil, gemessen nach der Paddle-Methode der U.S. Pharmacopoe XX bei 37 °C und 75 U/min, für das oder jedes Arzneimittel im wesentlichen wie folgt ist:

   a) 10 - 40 % Freisetzung innerhalb 1 h nach Verabreichung;
   b) 20 - 60 % Freisetzung innerhalb 4 h nach Verabreichung;
   c) 40 - 80 % Freisetzung innerhalb 8 h nach Verabreichung; und
   d) ≥ 80 % Freisetzung innerhalb 16 h nach Verabreichung.

14. Verfahren zur Herstellung von Mikrokapseln nach einem der Ansprüche 1-13, das die Schritte umfaßt:

   a) Auflösen oder Dispergieren eines Arzneimittels und eines biologisch abbaubaren Polymeren in einem Lösungsmittel unter Bildung eines Gemisches;
   b) Mikrofluidisieren des Gemisches in eine externe Phase unter Bildung einer Emulsion, in der die Emulsionströpfchen einen mittleren Durchmesser von weniger als 1 mm aufweisen; und
   c) Rühren der Emulsion unter Bildung von Mikrokapseln einer Größe (D 50 %) zwischen etwa 100 nm und 900 nm.

**Revendications**

1. Formulation pharmaceutique effervescente destinée à l'administration par voie orale, retardée et contrôlée, d'une quantité pharmaceutiquement efficace d'un médicament choisi parmi un agent bloquant les canaux du calcium, un inhibiteur de l'ACE, un analgésique narcotique ou des analogues ou combinaisons de ceux-ci, ladite formulation comprenant des microcapsules ayant un D 50 % compris entre 100 nm et 900 nm, le médicament étant inclus dans un polymère biodégradable, et le pH de la formulation étant ajusté de manière à optimiser la libération du médicament, la formulation étant adaptée à se disperser par suite d'addition d'eau pour former une boisson effervescente.

**2.** Formulation effervescente selon la revendication 1, dans laquelle le médicament est le diltiazem ou une combinaison du diltiazem et d'un analgésique narcotique et dans laquelle le pH de la formulation est supérieur à 7.

**3.** Formulation pharmaceutique effervescente selon la revendication 1 ou 2, dans laquelle la formulation est utilisée pour administrer le médicament à un patient une fois par jour, de façon à produire un effet thérapeutique pendant une durée essentiellement de 24 heures.

**4.** Formulation effervescente selon la revendication 1, dans laquelle le médicament est l'hydromorphone ou une combinaison d'hydromorphone et d'un agent bloquant les canaux du calcium, et dans laquelle le pH de la formulation est inférieur à 6 ou supérieur à 7.

**5.** Formulation pharmaceutique effervescente selon l'une quelconque des revendications précédentes, dans laquelle les microcapsules contiennent une charge de médicament comprise entre environ 10 % et 70 % en poids.

**6.** Formulation pharmaceutique effervescente selon l'une quelconque des revendications précédentes, dans laquelle les microcapsules ont un D 50 % compris entre environ 200 nm et 400 nm.

**7.** Formulation pharmaceutique effervescente selon l'une quelconque des revendications précédentes, dans laquelle les microcapsules contiennent une charge de médicament comprise entre environ 20 % et 50 % en poids.

**8.** Formulation pharmaceutique effervescente selon l'une quelconque des revendications précédentes, dans laquelle le médicament est choisi parmi le diltiazem, le vérapamil, la nifédipine, la nimodipine, la nicardipine, l'hydromorphone, le sulfate de codéine, l'oxycodone, le tartrate de dihydrocodéine, l'oxycodéinone, la morphine, le fentanyle, le sufentanile, l'oxymorphone, la buprénorphine, le captoprile, l'énalaprile, le lisonoprilc et leurs mélanges.

**9.** Formulation pharmaceutique effervescente selon la revendication 8, dans laquelle le médicament est un mélange de nifédipine et d'hydromorphone.

**10.** Formulation pharmaceutique selon l'une quelconque des revendications précédentes, dans laquelle la matrice polymère comprend le polylactide, le polyglycolide, le poly(acide lactique-co-acide glycolique), la poly($\varepsilon$-caprolactone), le poly(acide hydroxy-butyrique), des poly(ortho-esters), des polyacétals, des polydihydropyranes, des polycyanoacrylates, des polypeptides, des polypeptides réticulés et des stéréoisomères, des mélanges racémiques, des mélanges de copolymères et polymères de ces composés.

**11.** Formulation pharmaceutique selon la revendication 10, dans laquelle la matrice polymère comprend le poly-D,L-lactide.

**12.** Formulation pharmaceutique selon l'une quelconque des revendications précédentes, dans laquelle le schéma de libération, déterminé selon la méthode Paddle de la Pharmacopée US XX à 37°C et à 75 t/min pour le ou chaque médicament, se présente essentiellement comme suit :

a) 10-30 % de libération dans les 2 heures suivant l'administration ;
b) 30-60 % de libération dans les 4 heures suivant l'administration ;
c) 60-80 % de libération dans les 8 heures suivant l'administration ; et
d) $\geq$ 80 % de libération dans les 24 heures suivant l'administration.

**13.** Formulation pharmaceutique selon l'une quelconque des revendications 1-11, dans laquelle le schéma de libération, déterminé selon la méthode Paddle de la Pharmacopée US XX à 37°C et à 75 t/min pour le ou chaque médicament, se présente essentiellement comme suit :

a) 10-40 % de libération au cours de la première heure suivant l'administration ;
b) 20-60 % de libération dans les 4 heures suivant l'administration ;
c) 40-80 % de libération dans les 8 heures suivant l'administration ; et
d) $\geq$ 80 % de libération dans les 16 heures suivant l'administration.

**14.** Procédé de fabrication de microcapsules selon l'une quelconque des revendications 1-13, qui comprend les étapes consistant :

a) à dissoudre ou disperser un médicament et un polymère biodégradable dans un solvant pour former un mélange ;

b) à microfluidiser ledit mélange dans une phase externe pour former une émulsion dans laquelle les gouttelettes d'émulsion ont un diamètre moyen inférieur à 1 mm ; et

c) à agiter ladite émulsion pour former des microcapsules ayant une taille (D 50 %) comprise entre 100 nm et 900 nm.

FIG. 1

FIG. 2

FIG. 3

TIME    (HOURS)

—□— Db2

—◧— Db3

—◆— Db4

—◇— Db5

FIG. 4

FIG. 5

FIG. 6

Legend:
- □ pH1.2
- ◆ pH6.0
- □ pH7.4

FIG. 7

pH1.2
pH6.0
pH7.4

Figure 8

FIGURE 9

FIGURE 10